# FASCICULE DE BREVET EUROPEEN

(11) **EP 1 135 175 B1**
(45) Date de publication et mention de la délivrance du brevet: **14.07.2004**
(21) Numéro de dépôt: 99972934.6
(22) Date de dépôt: 09.11.1999
(51) Int. Cl.: A61L 15/32, C08H 1/06

(54) **PREPARATION D'UN MATERIAU COLLAGENIQUE AVEC UNE DEGRADATION IN VIVO CONTROLEE**
HERSTELLUNG VON KOLLAGENMATERIAL MIT IN VIVO KONTROLLIERTER BIOABBAUBARKEIT
METHOD FOR PREPARING A COLLAGEN MATERIAL WITH CONTROLLED IN VIVO DEGRADATION

(30) Priorité: 30.11.1998 FR 9815072
(43) Date de publication de la demande: 26.09.2001
(73) Titulaire: Imedex Biomateriaux, 69630 Chaponost (FR)
(72) Inventeur: BAYON, Yves, F-69100 Villeurbanne (FR); GRAVAGNA, Philippe, F-69540 Irigny (FR); TAYOT, Jean-Louis, F-69890 La Tour de Salvagny (FR)
(74) Mandataire: Bernasconi, Jean Raymond
(86) Numéro de dépôt international: PCT/FR1999/002746
(87) Numéro de publication internationale: WO 2000/032246

(56) Documents cités:
- EP-A- 0 351 296
- WO-A-95/34332
- US-A- 5 035 715
- US-A- 5 674 290
- US-A- 5 681 869

## Description

La présente invention concerne un procédé de préparation d'un matériau collagénique, permettant de contrôler la vitesse de biodégradation in vivo de ce matériau.

Elle est plus précisément relative à un procédé de traitement d'un composant collagénique permettant d'obtenir des matériaux de stabilité et de propriétés mécaniques variables selon les conditions dudit traitement, adaptés aux diverses applications biomédicales du collagène.

Les biomatériaux à base de collagène sont actuellement utilisés dans de multiples applications en présentant comme avantage majeur d'être résorbables. Cependant, selon leurs applications, il est nécessaire de contrôler leur dégradation biologique. En effet, les propriétés mécaniques du matériau collagénique implanté doivent progressivement diminuer et ledit matériau doit être finalement entièrement digéré sur un période déterminée.

Selon les applications de ces biomatériaux à base de collagène, la dégradation de ce dernier doit en général intervenir en un temps allant de quelques jours à plusieurs semaines.

Pour atteindre ces objectifs, les propriétés du collagène peuvent être modifiées de plusieurs façons possibles. On connaît ainsi dans la technique, des traitements conduisant à la formation de liaisons ioniques, de liaisons hydrogènes ou encore de liaisons covalentes (Chvapil et al., in International Review of Connective Tissue Research, Vol. 6, Eds. D.A. Hall and D.S. Jackson, Academic Press, UK, 1973,1-61).

La création de liens intermoléculaires permet d'augmenter le temps de biodégradation du matériau collagénique et la résistance mécanique des fibres de collagène tout en diminuant la capacité d'absorption d'eau, la solubilité et la vitesse de dégradation enzymatique de ces dernières (Pachence et al., Medical Device & Diagnostic Industry, 1987, 9, 46-55).

On a ainsi proposé dans la technique des procédés permettant de réticuler le collagène soit par des méthodes physiques soit par des méthodes chimiques.

Les méthodes chimiques font appel à des agents réticulants comme des composés aldéhydiques parmi lesquels on peut citer en particulier le formaldéhyde, le glutaraldéhyde, le succinaldéhyde, le glyoxal et l'acroléine ou encore les carbodiimides, les diisocyanates et les dérivés azide (Pachence et al., Medical Device & Diagnostic Industry, 1987, 9, 46-55 ; Weadock et al., Biomat. Med. Dev. Art. Org., 1983-84, 11, 293-318 ; BIOETICA et INSERM, FR 2 617 855).

Les composés aldéhydiques sont certes les agents réticulants les plus utilisés mais ils génèrent des biomatériaux potentiellement cytotoxiques.

Il est souhaitable d'introduire le moins possible de produits chimiques dans un biomatériau implantable, car ces additifs engendrent des complications et contraintes réglementaires de plus en plus lourdes pour démontrer l'absence de toxicité de tels produits.

On connaît par ailleurs dans la technique, un procédé pour modifier le collagène en formant des fonctions aldéhydes au sein même de celui-ci, par coupure oxydative à l'acide periodique ou l'un de ses sels, ce traitement permettant la réticulation du collagène à pH neutre ou basique (M. Tardy et J.L. Tayot, US 4 931 546).

Enfin, il a également été proposé dans la technique de modifier les propriétés du collagène en fonctionnalisant les groupes amino et carboxyles des amino-acides qu'il contient. Selon cette approche, des modifications de charge et de polarité peuvent ainsi ralentir ou accélérer la dégradation du collagène (Green et al., Biochem. J., 1953, 154, 181-187 ; Gustavson, Ark. Kemi., 1961, 55, 541-546).

Les méthodes physiques comprennent quant à elles la déshydratation, le vieillissement, le chauffage en absence d'humidité ou encore l'irradiation par les rayons ultraviolets, par rayonnement béta ou gamma.

Parmi ceux-ci, le traitement par irradiation au rayonnement béta ou gamma est utilisé pour stériliser les matériaux collagéniques deshydratés. mais conduit à des matériaux dont la résistance est difficilement prédictible à la lumière de la littérature existante.

Les différents paramètres pouvant intervenir dans ce type de traitement ne sont pas connus de manière suffisante pour permettre de contrôler la qualité des biomatériaux collagéniques résultants, en particulier du point de vue de leur résistance mécanique et de leur vitesse de biodégradation (Sintzel et al., Drug Dev. Ind. Pharm., 1997, 23, 857-878).

Le brevet US 5,035,715 décrit une irradiation par rayonnement gamma d'un mélange sensiblement non humide de collagène et d'un matériau minéral, ce qui permet d'obtenir une certaine réticulation.

Le brevet EP 0 351 296 décrit une irradiation gamma de billes de collagène permettant d'augmenter leur densité.

La demande WO 95/34332 décrit la siérilisation d'une prothèse valvulaire d'origine tissulaire porcine, contenant donc du collagène, par un faisceau d'électrons ou encore par irradiation X ou gamma. L'irradiation électronique de type béta de la prothèse préalablement légèrement réticulée par un agent chimique entraîne une dégradation moindre que l'irradiation gamma. Ce document n'enseigne pas qu'il est possible d'accroître la réticulation et la résistance à la dégradation d'une telle valve par rayonnement béta. Au contraire il enseigne que le rayonnement béta n'a guère d'influence sur la réticulation.

Le brevet US 5,674,290 décrit la stérilisation par irradiation gamma d'implants collagéniques ayant une teneur en eau élevée, dans une enveloppe étanche et transparente au rayonnement gamma. Lorsque cet enseignement est appliqué au collagène, le collagène est préalablement réticulé par un agent chimique. Ce document constate que, contrairement à la stérilisation par irradiation gamma d'un matériau collagénique sec, la stérilisation par rayonnement gamma du matériau collagénique humide ne modifie que très légèrement la dégradabilité enzymatique du maténau. Le document suggère, de façon erronée, que la stérilisation d'un tel matériau par faisceau d'électrons serait équivalente à la stérilisation par irradiation par stérilisation gamma.

On constate donc que les divers traitements que l'on connaît dans la technique permettant d'agir sur certaines propriétés des matériaux collagéniques, sont soit à l'origine de toxicités indésirables ou potentielles dans les applications envisagées, ou encore difficiles ou coûteux à mettre en oeuvre. ou ne permettent pas de contrôler efficacement les propriétés du matériau final obtenu.

La présente invention a pour objectif de fournir un traitement permettant d'obtenir des matériaux collagéniques dont la vitesse de dégradation in vivo et les propriétés mécaniques sont modulables suivant les applications potentielles desdits matériaux.

Un autre objectif de l'invention est de fournir un procédé de traitement conduisant à un biomatériau prêt à l'emploi, en procédant simultanément à la réticulation et à la stérilisation du matériau collagénique.

A cette fin, la présente invention a pour objet un procédé pour la préparation d'un matériau collagénique réticulé, biocompatible, non toxique et à vitesse de biodégradation in vivo contrôlée, caractérisé en ce qu'il comprend le fait de soumettre un composant collagénique à l'état humide à une irradiation par rayonnement béta, le matériau collagénique obtenu étant stérile et biodégradable en quelques jours à plusieurs semaines.

L'invention a aussi pour objet le procédé précité caractérisé en ce que le composant collagénique à l'état humide est associé à un réseau de fibres de collagène de préférence de structure hélicoïdale, préalablement à son irradiation.

L'invention concerne aussi les matériaux obtenus par le procédé ci-dessus.

Elle concerne encore un matériau bicomposite qui est biocompatible, non-toxique et stérile, à vitesse de biodégradation in vivo contrôlée, suturable ou agrafable, caractérisé en ce qu'il comprend uniquement ou principalement, deux couches intimement associées et réticulées avec interpénétration des réseaux réticulés, l'une desdites couches étant formée d'un film à base d'un composant collagénique réticulé et l'autre d'une compresse tassée formée de fibres de collagène réticulé rendues insolubles, notamment de collagène de structure hélicoïdale, préparées à partir de collagène solubilisé ou dispersé dans une solution aqueuse.

Les inventeurs ont découvert de manière tout à fait surprenante et imprévisible, que les propriétés de matériaux collagéniques dépendent du mode d'irradiation béta ou gamma du rayonnement.

Ils ont découvert en particulier que le taux d'hydratation du matériau irradié joue lui-même un rôle décisif dans les propriétés finales du matériau collagénique résultant.

Ils ont en effet observé que les résultats obtenus sont nettement différents suivant la nature du composant collagénique traité et suivant les conditions de stérilisation par irradiation appliquées.

Les inventeurs ont ainsi mis en évidence un traitement permettant à la fois la réticulation et la structuration du composant collagénique traité, simultanément à sa stérilisation, conduisant, à partir d'un matériau humide sensiblement dépourvu d'agent complémentaire de réticulation et, de préférence, non réticulé, à un matériau prêt à l'emploi à propriétés définies, en particulier du point de vue de sa résistance mécanique et de son temps de dégradation in vivo.

Selon l'invention, le terme "structuration" du composant collagénique se rapporte à la balance entre le taux de réticulation et le taux d'hydrolyse dudit composant collagénique qui conduit à des biomatériaux plus ou moins résistants.

Selon l'invention, la nature du composant collagénique se réfère en particulier à son état (taux d'humidité), son pH ou encore sa fonctionnalisation, le cas échéant.

Le composant coliagénique mis en oeuvre dans le procédé de l'invention se trouve à l'état humide. Par état humide, on entend une matière présentant un taux d'humidité supérieur à 30 %, plus préférentiellement compris entre 40 et 95 %.

A titre comparatif, l'état sec correspond quant à lui à une matière dont le taux d'humidité est inférieur à 30 %, celui-ci étant de préférence compris entre 5 et 20 %.

Dans ce cas, celui-ci peut se présenter, à l'état humide, sous la forme d'un gel ou d'une solution aqueuse, ou à l'état partiellement deshydrate. sous la forme d'un film. le taux d'humidité étant dans ce dernier cas voisin de 30 % à la différence des gels et solutions qui contiennent une beaucoup plus grande quantité d'eau.

Quelle que soit sa forme, la concentration en composant collagénique (matière sèche) est au minimum de 0,5 %, celle-ci étant de préférence supérieure à 2,5 %.

Le composant collagénique utilisé aux fins de l'invention peut consister en ou comprendre du collagène indifféremment d'origine animale ou humaine ou obtenu par des moyens de recombinaison génétique. On utilise de préférence du collagène natif, solubilisé à pH acide ou encore tel qu'obtenu après traitement par digestion à la pepsine. Il peut s'agir en particulier de collagène bovin de type I ou de collagènes humains de type I ou III ou encore de mélanges en toutes proportions de ces derniers.

Le composant collagénique peut également consister en ou comprendre du collagène modifié par coupure oxydative, notamment à l'aide d'acide periodique ou l'un de ses sels selon la technique précitée.

On rappelle brièvement que cette technique consiste à soumettre une solution acide de collagène à l'action de l'acide periodique ou l'un de ses sels par mélange avec une solution de cet acide ou sel à une concentration comprise entre 1 et 10⁻⁵ M, de préférence entre 5 10⁻³ M et 10⁻¹ M à une température voisine de la température ambiante, pendant une durée pouvant aller de 10 minutes à 72 heures.

Selon l'invention, on utilise une solution aqueuse acide de collagène dont la concentration est comprise entre 5 et 50 g/l, celle-ci étant de préférence de 30 g/l.

Ce traitement provoque des coupures dans certains constituants du collagène qui sont l'hydroxylysine et les sucres et crée ainsi des sites réactifs sans en provoquer la réticulation tant que le pH reste acide.

La coupure oxydative du collagène a comme fonction de permettre une réticulation modérée ultérieure du matériau collagénique mais l'invention n'exclut pas que l'on puisse réaliser cette fonction par d'autres moyens de réticulation modérée, par exemple par rayonnement béta ou gamma, ou d'autres agents de réticulation modérée, par exemple des agents chimiques à des doses suffisamment faibles et non toxiques.

Le composant collagénique mis en oeuvre selon l'invention peut encore consister en ou comprendre du collagene ayant perdu, au moins partiellement, sa structure hélicoïdale, notamment par chauffage à une température supérieure à 37°C, de préférence comprise entre 40 et 50°C pendant moins d'une heure.

On peut obtenir notamment une préparation finale que l'on peut assimiler à de la gélatine mais dont le poids moléculaire des chaînes élémentaires est supérieur ou égal à 100 kDa.

Le traitement par chauffage à température supérieure à 37°C de la solution de collagène entraîne la perte progressive de la structure hélicoïdale du collagène, mais l'invention n'exclut pas que l'on puisse réaliser cette fonction par d'autres moyens physiques ou chimiques, par exemple par ultra-sonication, ou par addition d'agents chaotropiques.

Le composant collagénique peut aussi être formé de ou comprendre du collagène fonctionnalisé au niveau des fonctions amino et/ou carboxyle des amino-acides par exemple par succinylation, méthylation ou encore par greffage d'acides gras, ou toute autre méthode connue pour modifier chimiquement le collagène.

L'invention s'applique également à des mélanges en toutes proportions des différents composants collagéniques précités.

Le composant collagénique selon l'invention peut également contenir un additif hydrophile macromoléculaire.

Conformément à la présente invention, l'additif hydrophile macromoléculaire présente un poids moléculaire avantageusement supérieur à 3 000 daltons.

Il peut s'agir de polymères hydrophiles de synthèse. avantageusement de poids moléculaire compris entre 3 000 et 20 000 daltons Le polyéthylène glycol est particulièrement préféré.

Il peut s'agir également de polysaccharides, parmi lesquels on peut citer l'amidon, le dextrane et la cellulose qui sont préférés.

On peut également prévoir l'utilisation de tels polysaccharides sous forme oxydée faisant apparaître des fonctions carboxyliques dans ces molécules.

Les mucopolysaccharides peuvent également convenir aux fins de l'invention mais ne sont pas préférés car leur origine animale particulière les rend difficiles à préparer en satisfaisant aux normes réglementaires de traçabilité.

L'additif hydrophile est sélectionné en fonction de divers paramètres liés notamment à son application, comme son prix, son innocuité. sa biodégradabilité et/ou sa facilité d'élimination, notamment par voie rénale, en cas d'application thérapeutique.

La concentration en additif hydrophile est de 2 à 10 fois inférieure à celle du composant collagénique.

On décrit plus précisément ci-après le procédé de préparation d'un matériau collagénique selon la présente invention.

Celui-ci comprend une étape selon laquelle le composant collagénique tel que défini ci-dessus, est soumis à une irradiation par rayonnement béta de doses variables selon la résistance mécanique du biomatériau final souhaitée ainsi que sa vitesse de biodégradation in vivo.

Le composant collagénique est avantageusement traité à pH neutre, de préférence compris entre 6,5 et 8 dans le but de favoriser les réactions de réticulation et d'obtenir un biomatériau biocompatible grâce au pH physiologique.

Le composant collagénique est réticulé/structuré par irradiation aux rayons béta à des doses stérilisantes, avantageusement de l'ordre de 5 à 50 KGrey, de préférence comprise entre 25 et 35 KGrey.

Dans certaines conditions, les doses peuvent être réduites, par exemple jusqu'à 5 KGrey pour des matériaux déjà stériles ou très peu contaminés, ce qui permet de moduler à la baisse le degré de réticulation.

Conformément à l'invention, le traitement par irradiation béta appliquée à un composant collagénique à l'état humide permet d'obtenir un matériau présentant une forte résistance à la dégradation qui est ainsi biodégradable in vivo en plusieurs semaines alors qu'une exposition à un rayonnement gamma, conduit à un biomatériau conduisant à une faible résistance à la dégradation qui sera ainsi biodégradable in vivo en quelques jours.

Ces résultats sont inverses de ceux obtenus pour un composant collagénique à l'état sec pour lequel une irradiation au rayonnement béta conduit à un biomatériau dont la dégradation sera obtenue en quelques jours alors que l'irradiation gamma conduit à un biomatériau dont la dégradation sera obtenue en plusieurs semaines.

Selon une variante de réalisation de l'invention, le composant collagénique destiné à être structuré par irradiation est associé au préalable à un réseau de fibres de collagène non dénaturé qui est avantageusement mis sous forme d'une compresse tassée.

La préparation de cette compresse peut être réalisée à partir de collagène natif, oxydé à l'aide d'acide périodique ou l'un de ses sels.

Des fibres sont formées à partir de la solution résultante qui sont ensuite réticulées par neutralisation.

Les fibres de collagène oxydé, de structure hélicoïdale, ainsi réticulées sont lyophilisées et deshydratées puis tassées pour former la compresse.

On dépose ensuite sur cette compresse tassée de collagène fibreux, une solution de composant collagénique préparée comme indiquée précédemment.

L'ensemble est avantageusement séché et partiellement réhydraté pour augmenter la concentration en composant collagénique.

On procède ensuite au traitement par irradiation comme décrit ci-dessus.

On obtient ainsi la structuration/réticulation de l'ensemble précité conduisant à un matériau bicomposite comprenant une couche formant film à base d'un composant collagénique réticulé associée à une compresse tassée de fibres de collagène réticulé avec interpénétration des réseaux réticulés.

D'une manière générale, ce procédé peut être appliqué à des compresses de fibres. tissées ou non tissées, de collagène avantageusement de structure hélicoïdale.

Les matériaux collagéniques obtenus par le procédé de l'invention sont utiles pour la prévention des adhérences post-opératoires et/ou la cicatrisation de plaies.

De tels matériaux sont particulièrement utiles pour favoriser la cicatrisation des plaies cutanées et des plaies chirurgicales. Leur nature biocompatible les rend très facilement colonisables par les cellules des différents tissus au contact desquels ils sont appliqués.

Dans le cas des plaies internes, il a été observé que cette cicatrisation s'effectue de manière harmonieuse sans entraîner de proliférations tissulaires fibreuses anarchiques responsables d'adhérences post-opératoires.

L'activité cicatrisante de ces matériaux peut évidemment être renforcée par l'addition de facteurs de croissance ou de différenciation cellulaires.

Les matériaux selon l'invention sont donc recommandés pour assurer rapidement une cicatrisation de qualité, aussi proche que possible de l'anatomie initiale.

Les matériaux collagéniques associés à un réseau de fibres de collagène sont également utiles pour la cicatrisation de plaies comme indiqué auparavant. Ils présentent l'avantage d'être suturables et agrafables compte tenu de leur résistance très élevée résultant de l'irradiation aux rayonnements béta.

Ces matériaux peuvent également être utilisés pour le remplacement de tissus ou parois (par exemple paroi oesophagienne, intestinale...) ou pour le comblement de ceux-ci (en cas d'ablation partielle d'une partie de tissu ou paroi).

### EXEMPLES

### EXEMPLE 1 : Structuration d'un gel de collagène chauffé.

Une solution acide de collagène bovin de type I, à la concentration de 16 %, est préparée en solubilisant la poudre acide de collagène dans de l'eau ultrafiltrée déminéralisée, à une température comprise entre 40 et 60°C. pendant une durée inférieure à 30 minutes. La solution est neutralisée jusqu'à pH 7,45 par addition de soude normale, dès que la solution de collagène est suffisamment fluide, soit 5 à 10 minutes après l'addition de collagène dans l'eau, pour éviter l'hydrolyse du collagène.

La solution de collagène est ensuite filtrée stérilement sur une membrane de porosité 0,22 µm et conservée à une température comprise entre 0 et 10°C, avant son utilisation.

On obtient une préparation finale que l'on peut assimiler à de la gélatine, mais dont le poids moléculaire des chaînes élémentaires est supérieur ou égal à environ 100 kDa.

D'autres sources de collagène, connues de l'homme de l'art, peuvent être utilisées pour obtenir du collagène chauffé comme décrit ci-dessus dans cet exemple.

Dans le cas du collagène bovin de type 1, il peut être acido-soluble (extrait à partir de peaux ou de tendons à pH acide), ou solubilisé par digestion avec la pepsine, qui facilite les filtrations stérilisantes ultérieures du collagène chauffé.

La solution de collagène chauffé obtenue est ensuite mise en forme par moulage, "prilling" (formation de billes par passage dans des capillaires et recueillement des gouttes), séchage partiel (qui permet d'augmenter la concentration en composant collagénique), ou tout autre procédé à une température comprise entre 40 et 70°C, puis refroidie à une température comprise entre 0 et 25°C.

Ces traitements ont pour but d'obtenir des biomatériaux de toutes formes, adaptées aux applications recherchées, comme par exemple des tubes capillaires, des billes, microbilles, capsules, films, pouvant contenir des molécules d'intérêt thérapeutique.

Le biomatériau préparé est réticulé et stérilisé soit par irradiation gamma soit par irradiation béta, à la dose de 25 à 35 KGy.

Le biomatériau irradié par les rayonnements gamma se dissout dans l'eau déminéralisée, à 37°C, en moins de 24 heures.

Par contre, le traitement par irradiation béta réticule fortement le biomatériau. Celui-ci ne se dégrade pas à 60°C, dans l'eau déminéralisée. même après une journée. Sa capacité d'absorption d'eau est réduite, voire nulle. Elle est inférieure à 10 % du poids du biomatériau. Sa biodégradation est lente in vivo, et nécessite plusieurs semaines lorsque le site d'implantation du biomatériau, chez l'animal, est sous-cutané ou au contact des viscères.

Selon une variante, ce matériau peut être préparé à partir d'une solution de collagène natif de concentration comprise entre 0,5 et 3%, contenant 5 g/l de NaCl et non soumise à l'étape de chauffage, de manière à préserver la structure hélicoïdale du collagène.

### EXEMPLE 2 : Structuration d'un film de collagène partiellement réhydraté.

Le matériau de l'exemple 1 est préparé sous forme de film d'épaisseur 2 mm, sans aucun additif chimique.

Avant l'étape de traitement final par irradiation, le film obtenu est deshydraté sous un flux d'air sec et filtré pendant 24 heures.

Le film sec obtenu est ensuite réhydraté pendant 5 à 10 minutes dans de l'eau distillée.

Le film partiellement réhydraté est conditionné dans un emballage étanche à la vapeur d'eau, puis soumis au traitement par irradiation béta ou gamma à une dose de 25 à 35 kiloGrey.

Le film obtenu en final mesure entre 0,5 et 1 mm d'épaisseur en fonction du temps de réhydratation précédemment appliqué. Sa teneur en collagène est comprise entre 30 et 60%.

Le matériau stérilisé par irradiation béta est très résistant à des forces d'étirement et se dégrade in vivo, par voie sous-cutanée, en plus de 3 semaines.

Le matériau stérilisé par irradiation gamma est peu résistant à des forces d'étirement, gonfle spontanément au contact de l'eau à 37°C et se dégrade in vivo après implantation en moins d'une semaine.

En variante, ce matériau peut être préparé à partir d'une solution de collagène natif de concentration comprise entre 0.5 et 3 %, contenant 5 g/l de NaCl et non soumise à l'étape de chauffage, de manière à préserver la structure hélicoïdale du collagène.

### EXEMPLE 3 : Réalisation d'un matériau suturable par association du matériau collagénique de l'exemple 2 à un réseau de fibres de collagène de structure hélicoïdale.

### a) Préparation du réseau de fibres de collagène.

On prépare une solution acide de collagène bovin de type I à la concentration de 1 % en solubilisant la poudre acide dans de l'eau ultrafiltrée, déminéralisée à 20°C.

Cette solution est additionnée d'acide orthopériodique, à l'abri de la lumière, afin d'obtenir une concentration finale de 5 x 10⁻³ M en vue de préparer des fibres de collagène oxydé, conformément au brevet FR 2 601 371.

Après 2 heures de traitement, cette solution est injectée dans une série de capillaires parallèles, de diamètre intérieur voisin de 300 microns, qui produisent ainsi des tubes fins de collagène. coupés par unités de 4 cm de longueur, grâce à un dispositif de section automatique. Ces fins capillaires de collagène sont recueillis en vrac dans une solution tampon alcaline de carbonate de sodium 0,05 M à pH 9,3, additionnée d'éthanol à 30%.

Dans ces conditions, le collagène oxydé se réticule immédiatement et les tubes de collagène deviennent insolubles. Les tubes ainsi réticulés sont lavés dans des bains successifs d'eau ultrafiltrée. Une suspension de ces fibres est ensuite réalisée contenant 2 % de matière sèche collagénique, additionnée de glycérine à 1 % et étalée en plaques d'épaisseur 5 mm.

Les plaques sont ensuite lyophilisées et les compresses deshydratées sont tassées par pression, pour obtenir une épaisseur de 1 à 2 mm.

### b) Préparation de la solution de collagène chauffée.

On réalise une solution de collagène à 5 % chauffée dans les conditions décrites de l'exemple 1 ou 2.

### c) Association des deux matériaux précédents.

La solution préparée en "b)" est coulée sur la compresse tassée décrite au paragraphe "a)", à une dose de 0,2 ml/cm² environ.

L'ensemble est ensuite séché sous flux laminaire stérile pendant 24 heures.

Le matériau composite est ensuite partiellement réhydraté par trempage en eau, pendant 10 minutes.

Le matériau est conditionné dans un emballage étanche à la vapeur d'eau et stérilisé par irradiation béta (faisceau d'électrons accélérés).

On obtient un matériau extrêmement résistant à l'étirement, dont les propriétés mécaniques le rendent utilisable avec les techniques chirurgicales de suture et d'agrafage.

Ce matériau est constitué uniquement de collagène, sans aucun additif chimique. Il est non toxique, parfaitement toléré par l'organisme et peut être utilisé comme substitut des tissus animaux, actuellement utilisés par les chirurgiens (fascia tata, dure mère, etc.) dans les applications de remplacement ou de comblement de tissus, parois... ou encore de cicatrisation.

### EXEMPLE 4 : Structuration d'hydrocolloïdes collagéniques constitués de fibres de collagène natif oxydé.

Les hydrocolloïdes collagéniques sont réalisés à partir de collagène bovin natif, non digéré par la pepsine, et oxydé par l'acide periodique. suivant le procédé décrit dans le brevet US 4 931 546.

On emploie des fibres de collagène natif de type I extrait de peaux de jeunes veaux. D'autres sources de collagène, connues de l'homme de l'art. peuvent être utilisées.

Une solution de collagène est préparée à 30 g/l par mise en suspension en HCl 0,01 N, dans un volume de 720 ml. Elle est additionnée d'acide periodique à la concentration finale de 8 mM, soit 1,83 g/l.

L'oxydation est effectuée à une température de 22 ± 3°C, pendant 3 h ± 30 minutes, sous agitation et à l'abri de la lumière.

Puis, le collagène oxydé est précipité en ajoutant du NaCl 240 g/l. jusqu'à obtenir une concentration finale en NaCl de 41 g/l.

Après 30 minutes d'attente, le précipité est récolté par décantation à travers un tamis de toile de porosité comprise entre 20 et 100 µm, puis lave 4 fois avec une solution de NaCl 41 g/l en HCl 0,01 N pour éliminer toutes traces d'acide periodique ou de ses dérivés formés au cours de l'oxydation du collagène. On obtient un précipité de 110 g.

Puis, le chlorure de sodium est éliminé par trois lavages successifs du précipité salin de collagène, en utilisant un mélange acétone/eau (80/20, m/m).

Un lavage final en acétone 100 % permet d'obtenir 51,5 g d'un précipité acétonique, concentré, de collagène oxydé acide, non réticulé et dépourvu de produits toxiques, dus à l'emploi de l'acide périodique.

Le précipité acétonique est repris dans 390 ml d'eau déminéralisée ultrafiltrée (apyrogène) à 40°C pendant 5 à 10 minutes, pour obtenir une concentration en collagène de 4,1 %. Puis, le mélange est chauffé 30 minutes à 50 ± 5°C.

Avant utilisation, la solution de collagène chauffé oxydé peut être conservée à -20°C.

Pour la réalisation de l'hydrocolloïde, le collagène oxydé chauffé, comme préparé ci-dessus, à la concentration finale de 4,1 % est additionné, à 38°C, d'une solution stérile de PEG 4000 (polyéthylène glycol de poids moléculaire de 4 000) pour atteindre la concentration finale de 1,3 % de PEG et de 3,9 % en collagène total. Le pH de cette solution est ensuite neutralisé à 7,3.

Le mélange de collagène est coulé dans des supports hydrophobes, en polystyrène ou PVC, à raison de 0,2 g par cm². Il est évaporé, sous flux d'air stérile, pendant 18 à 24 heures, à une température voisine de 22°C. Le film résultant est réhydraté pendant 15 à 20 minutes dans de l'eau déminéralisée apyrogène, pour obtenir un hydrocolloïde. Ce biomatériau est structuré et stérilisé par irradiation au rayonnement béta ou gamma, à une dose comprise entre 25 et 35 KGy. Son humidité, avant stérilisation est comprise de préférence entre 75 et 95 %.

Les hydrocolloïdes structurés par irradiation au rayonnement gamma se délitent en moins de 24 heures, à 37°C, lorsqu'ils sont plongés dans de l'eau déminéralisée apyrogène.

Par contre, l'irradiation béta, aux doses mentionnées ci-dessus, permet d'obtenir un hydrocolloïde plus résistant qui garde son intégrité, même après avoir été plongé dans de l'eau déminéralisée apyrogène, à 37°C, pendant 24 heures.

### EXEMPLE 5 : Structuration d'hydrocolloïdes collagéniques constitués de fibres de collagène natif non oxydé.

Les hydrocolloïdes décrits dans cet exemple représentent une variante de l'exemple 4.

Ils sont préparés à partir de collagène chauffé non oxydé et de PEG 4000 comme additif hydrophile macromoléculaire.

On reprend du collagène I bovin natif acide, à la concentration finale de 3 %, dans de l'eau déminéralisée ultrafiltrée contenant 1 % de PEG 4000. Le mélange est maintenu à 42°C, sous agitation, jusqu'à obtenir une solution homogène, c'est-à-dire en moins de 30 minutes. Puis, il est dégazé sous vide.

Ce mélange est, ensuite, coulé dans des supports hydrophobes, en polystyrène ou PVC, à raison de 0,27 g par cm².

Il est séché, sous flux d'air stérile, pendant 18 à 24 heures.

Le film résultant est réhydraté, pendant 15 à 20 minutes, dans un tampon physiologique, afin d'obtenir un film de pH neutre, compris entre 7 et 8. On peut utiliser à cet effet un tampon phosphate 150 mM, de pH 8,2.

Ce biomatériau est structuré et stérilisé par irradiation au rayonnement béta ou gamma, à une dose comprise entre 25 et 35 KGy. Son humidité, avant stérilisation est comprise de préférence entre 75 et 95%.

Les propriétés des matériaux obtenus sont comparables aux matériaux décrits dans les exemples précédents. La stérilisation par irradiation béta rend plus résistant l'hydrocolloïde à base de collagène 1 bovin natif non oxydé que la stérilisation par irradiation gamma.

### EXEMPLE 6 : Structuration d'hydrocolloïdes collagéniques constitués de collagène I bovin pepsiné oxydé.

Les hydrocolloïdes décrits dans cet exemple représentent une variante des exemples 4 et 5.

On utilise du collagène I bovin pepsmé. On peut, de la même manière, utiliser des collagènes humains de type I ou III, ou leur mélange en toutes proportions.

Le collagène est oxydé comme décrit dans l'exemple 4 avec les modifications suivantes :

Le précipité acétonique de collagène oxydé est repris à la concentration finale de 3 %, dans de l'eau déminéralisée ultrafiltrée, à 40°C. Il est ensuite chauffé à 50°C, pendant 30 minutes. La solution de collagène chauffé oxydé est stérilisée par filtration sur membrane de porosité 0,45 µm, dans une étuve à 40°C.

A la température de 38°C, on ajoute au collagène oxydé comme préparé ci-dessus du PEG 4000 en solution aqueuse à 20%, pour atteindre la concentration finale de PEG de 1%. Le mélange est neutralisé jusqu'à un pH de 7,0, avec de la soude 0,5 N et 0,1 N. Il est ensuite réparti dans des supports hydrophobes, en polystyrène ou PVC, à raison de 0,27 g par cm².

Il est séché, sous flux d'air stérile, pendant 18 à 24 heures, à une température voisine de 22°C. Puis, il est réhydraté dans de l'eau déminéralisée ultrafiltrée.

Ce biomatériau est structuré et stérilisé par irradiation au rayonnement béta ou gamma, à une dose comprise entre 25 et 35 KGy.

Son humidité, avant stérilisation, est comprise de préférence entre 75 et 95%.

Comme pour les exemples précédents. réalisés à partir de biomatériaux humides, l'irradiation béta permet d'obtenir des matériaux plus résistants que l'irradiation gamma.

### EXEMPLE 7 : Structuration d'hydrocolloïdes collagéniques constitués de collagène de type I, pepsiné, chauffé, non oxydé.

Les hydrocolloïdes décrits dans cet exemple représentent une variante des exemples 4, 5 et 6.

Les hydrocolloïdes collagéniques sont réalisés à partir de collagène bovin natif de type I, digéré par la pepsine et chauffé.

On peut, de la même manière, utiliser des collagènes chauffés humains de type I, III ou leur mélange en toutes proportions.

On emploie une solution de collagène chauffé à 3 % et neutralisé, à pH 7,45, préparé suivant l'exemple 1.

On ajoute, à 42°C, au collagène chauffé à 3 %, une solution stérile concentrée de PEG 4000 pour obtenir une concentration finale de 0,9 % en PEG et de 2,7 % de collagène. Le pH de la solution est ajusté à 7,0, par addition d'une solution concentrée d'hydroxyde de sodium.

Ce mélange est ensuite coulé dans des supports hydrophobes. en polystyrène ou PVC, à raison de 0,27 g par cm².

Il est séché, sous flux d'air stérile, pendant 18 à 24 heures, à une température voisine de 22°C.

Le film résultant est réhydraté, pendant 15 à 20 minutes, dans de l'eau déminéralisée ultrafiltrée.

Ce biomatériau est structuré et stérilisé par irradiation béta ou gamma, à une dose comprise entre 25 et 35 KGy.

Son humidité, avant stérilisation, est comprise de préférence entre 75 et 95 %.

Comme pour les exemples précédents, réalisés à partir de biomatériaux humides, la gamma-irradiation permet d'obtenir des matériaux moins résistants que l'irradiation béta.

### EXEMPLE COMPARATIF : Structuration d'un film de collagène sec.

On réalise un film de collagène comme décrit dans la demande de brevet FR 9711589.

On emploie du collagène oxydé chauffé, préparé suivant l'exemple **6**[7].

Le collagène utilisé comme matière première pour la préparation du collagène oxydé chauffé est du collagène bovin de type I, éventuellement solubilisé par digestion à la pepsine et, purifié par des précipitations salines. selon les techniques déjà décrites. On peut utiliser, de la même manière, des collagènes humains de type I ou III ou leur mélange en toutes proportions.

Une solution de collagène chauffé oxydé à 3 % est additionnée a 35°C, d'une solution stérile concentrée de PEG 4000 et de glycérine, pour obtenir une concentration finale de 0,9 % en PEG, de 0,54 % en glycérine et de 2,7% en collagène total. Le pH de la solution est ajusté à 7,0, par addition d'une solution concentrée d'hydroxyde de sodium.

Cette solution est coulée en couche mince à densité de 0,133 g/cm² sur un support plan hydrophobe de type PVC ou polystyrène. Le film est séché sous flux d'air stérile, à une température voisine de 22°C.

L'épaisseur moyenne du film obtenu est de 40 à 50 µm, avec un taux moyen d'humidité de 10 %.

Le film sec est irradié par rayonnement béta ou gamma à une dose comprise entre 25 et 35 KGy.

Dans ce cas, le film sec irradié par rayonnement gamma présente des propriétés mécaniques supérieures au film irradié par rayonnement béta comme l'indique le taux de gonflement, plus faible des films traités par les rayons gamma. Parallèlement, la biodégradation du film sec traité par les rayons gamma, in vivo, est supérieure à 3 semaines. Par contre, le film sec irradié par rayonnement béta est "digéré" in vivo en moins d'une semaine.

| **Mode irradiation** | **Résistance à la dégradation, en milieu acide (pH 2), à 37°C** | **Biodégradation in vivo** |
|---|---|---|
| **Béta 25-35 KGy** | | |
| (faisceau d'électrons accélérés) | | |
| Etat sec | faible | quelques jours |
| Etat humide | forte | plusieurs semaines |

| **Gamma 25-35 KGy** | | |
|---|---|---|
| (source radioactive) | | |
| Etat sec | forte | plusieurs semaines |
| Etat humide | faible | quelques jours |

## Revendications

1. Procédé pour la préparation d'un matériau collagénique réticulé, biocompatible, non toxique et à vitesse de biodégradation in vivo contrôlée, **caractérisé en ce qu'**il comprend le fait de soumettre un composant collagénique sensiblement dépourvu d'agent complémentaire de réticulation, et de préférence non réticulé, à l'état humide à une irradiation par rayonnement béta, le matériau collagénique obtenu étant stérile et biodégradable en quelques jours à plusieurs semaines.

2. Procédé selon la revendication 1, **caractérisé en ce que** le composé collagénique présente un taux d'humidité supérieur à 30 %, de préférence supérieur à 40 %.

3. Procédé selon l'une quelconque des revendications 1 et 2, **caractérisé en ce que** le composant collagénique est sous forme d'un gel.

4. Procédé selon l'une quelconque des revendications 1 et 2. **caractérisé en ce que** le composant collagénique est sous forme d'une solution aqueuse.

5. Procédé selon l'une quelconque des revendications précédentes, **caractérisé en ce que** le composant collagénique présente un pH neutre, de préférence compris entre 6,5 et 8.

6. Procédé selon l'une quelconque des revendications précédentes, **caractérisé en ce que** la concentration en composant collagénique (matière sèche) est de 0,5 % au minimum et de préférence supérieure à 2,5 %.

7. Procédé selon l'une quelconque des revendications précédentes, **caractérisé en ce que** le composant collagénique consiste en ou comprend du collagène ayant perdu au moins partiellement sa structure hélicoïdale.

8. Procédé selon la revendication 7, **caractérisé en ce que** le collagène ayant perdu au moins partiellement sa structure hélicoïdale est formé de collagène non hydrolysé, constitué majoritairement de chaînes α.

9. Procédé selon l'une quelconque des revendications 7 et 8, **caractérisé en ce que** le composant collagénique consiste en ou comprend du collagène ayant perdu au moins partiellement sa structure hélicoïdale par chauffage au-dessus de 37°C, de préférence comprise entre 40 et 50°C.

10. Procédé selon l'une quelconque des revendications 1 à 6. **caractérisé en ce que** le composant collagénique consiste en ou comprend du collagène oxydé.

11. Procédé selon la revendication 10, **caractérisé en ce que** le collagène oxydé consiste en du collagène modifié par coupure oxydative à l'aide d'acide periodique ou l'un de ses sels.

12. Procédé selon l'une quelconque des revendications 1 à 6. **caractérisé en ce que** le composant collagénique comprend du collagène fonctionnalisé au niveau des fonctions amino et/ou carboxyle des amino-acides.

13. Procédé selon l'une quelconque des revendications précédentes, **caractérisé en ce que** le composant collagénique comprend un additif hydrophile macromoléculaire.

14. Procédé selon la revendication 12, **caractérisé en ce que** l'additif hydrophile macromoléculaire présente un poids moléculaire supérieur à 3 000 Daltons.

15. Procédé selon l'une quelconque des revendications 13 et 14, **caractérisé en ce que** l'additif hydrophile macromoléculaire est un polymère hydrophile de poids moléculaire compris entre 3 000 et 20 000 Daltons.

16. Procédé selon l'une quelconque des revendications 13 à 15, **caractérisé en ce que** l'additif hydrophile macromoléculaire est le polyéthylèneglycol.

17. Procédé selon l'une quelconque des revendications 13 à 16, **caractérisé en ce que** l'additif hydrophile macromoléculaire est choisi parmi les polysaccharides notamment l'amidon, le dextrane ou la cellulose, ou les mucopolysaccharides.

18. Procédé selon la revendication 17, **caractérisé en ce que** l'additif hydrophile macromoléculaire est un polysaccharide sous forme oxydée.

19. Procédé selon l'une quelconque des revendications précédentes, **caractérisé en ce que** le composant collagénique est irradié à une dose de 5 à 50 KGy.

20. Procédé selon l'une quelconque des revendications précédentes, **caractérisé en ce que** le composant collagénique est irradié à une dose de 20 à 50 KGy, de préférence 25 à 35 KGy

21. Procédé selon l'une quelconque des revendications précédentes, **caractérisé en ce que** le composant collagénique est irradié par rayonnement béta, le matériau collagénique résultant étant fortement réticulé et biodégradable in vivo en plusieurs semaines.

22. Procédé selon l'une quelconque des revendications précédentes, **caractérisé en ce que** le composant collagénique à l'état humide est associé à un réseau de fibres de collagène de préférence de structure hélicoïdale, préalablement à son irradiation.

23. Procédé selon la revendication 22, **caractérisé en ce que** le réseau de fibres de collagène consiste en une compresse de fibres tassées obtenue à partir d'une solution acide de collagène natif, par traitement à l'aide d'acide périodique ou l'un de ses sels, formation de fibres puis réticulation par neutralisation, les fibres de collagènes de structure hélicoïdale réticulées résultantes étant tassées sous pression, et **en ce qu'**on dépose une solution de composant collagénique sur ladite compresse puis **en ce que** l'ensemble est irradié aux rayonnements béta.

24. Matériau collagénique bicomposite qui est biocompatible. non-toxique et stérile, à vitesse de biodégradation in vivo contrôlée, suturable ou agrafable, **caractérisé en ce qu'**il comprend uniquement ou principalement. deux couches intimement associées et réticulées avec interpénétration des réseaux réticulés, l'une desdites couches étant formée d'un film à base d'un composant collagénique réticulé et l'autre d'une compresse tassée formée de fibres de collagène réticulé rendues insolubles, notamment de collagène de structure hélicoïdale, préparées à partir de collagène solubilisé ou dispersé dans une solution aqueuse.

25. Matériau collagénique bicomposite selon la revendication 24, **caractérisé en ce qu'**il est réticulé par le procédé selon l'une quelconque des revendications 22 et 23.

26. Matériau collagénique selon l'une quelconque des revendications 24 et 25, **caractérisé en ce que** le composant collagénique est tel que défini selon l'une quelconque des revendications 7 à 18.

## Patentansprüche

1. Verfahren zur Herstellung eines nicht-toxischen, biologisch verträglichen vernetzten Collagenmaterials mit *in vivo* kontrollierbarer biologischer Abbaugeschwindigkeit, **dadurch gekennzeichnet, dass** in ihm eine Collagenkomponente, die im Wesentlichen frei von einem komplementären Vernetzungsmittel und vorzugsweise unvernetzt ist, im feuchten Zustand einer Bestrahlung mit β-Strahlung unterworfen wird, wobei das erhaltene Collagenmaterial steril und innerhalb von einigen Tagen bis mehreren Wochen biologisch abbaubar ist.

2. Verfahren nach Anspruch 1, **dadurch gekennzeichnet, dass** der Feuchtigkeitsgehalt der Collagenverbindung mehr als 30 % und vorzugsweise als 40 % beträgt.

3. Verfahren nach Anspruch 1 oder 2, **dadurch gekennzeichnet, dass** die Collagenkomponente in Form eines Gels vorliegt.

4. Verfahren nach Anspruch 1 oder 2, **dadurch gekennzeichnet, dass** die Collagenkomponente in Form einer wässrigen Lösung vorliegt.

5. Verfahren nach einem der vorhergehenden Ansprüche, **dadurch gekennzeichnet, dass** der pH-Wert der Collagenkomponente neutral ist und vorzugsweise 6,5 bis 8 beträgt.

6. Verfahren nach einem der vorhergehenden Ansprüche, **dadurch gekennzeichnet, dass** die Konzentration der Collagenkomponente (Trockensubstanz) mindestens 0,5 % und vorzugsweise mehr als 2,5 % beträgt.

7. Verfahren nach einem der vorhergehenden Ansprüche, **dadurch gekennzeichnet, dass** die Collagenkomponente aus Collagen, das seine Helixstruktur wenigstens teilweise verloren hat, besteht oder dieses enthält.

8. Verfahren nach Anspruch 7, **dadurch gekennzeichnet, dass** das Collagen, das seine Helixstruktur wenigstens teilweise verloren hat, von nicht hydrolysiertem Collagen gebildet wird, das überwiegend aus α-Ketten besteht.

9. Verfahren nach Anspruch 7 oder 8, **dadurch gekennzeichnet, dass** die Collagenkomponente aus Collagen, das seine Helixstruktur durch Erwärmen auf über 37°C und vorzugsweise auf 40 bis 50 °C wenigstens teilweise verloren hat, besteht oder dieses enthält.

10. Verfahren nach einem der Ansprüche 1 bis 6, **dadurch gekennzeichnet, dass** die Collagenkomponente aus oxidiertem Collagen besteht oder dieses enthält.

11. Verfahren nach Anspruch 10, **dadurch gekennzeichnet, dass** das oxidierte Collagen aus Collagen besteht, das durch oxidatives Schneiden mit Periodsäure oder einem ihrer Salze modifiziert worden ist.

12. Verfahren nach einem der Ansprüche 1 bis 6, **dadurch gekennzeichnet, dass** die Collagenkomponente Collagen umfasst, dessen Amino- und/oder Carboxylfunktionen der Aminosäuren funktionalisiert worden sind.

13. Verfahren nach einem der vorhergehenden Ansprüche, **dadurch gekennzeichnet, dass** die Collagenkomponente ein hydrophiles makromolekulares Additiv umfasst.

14. Verfahren nach Anspruch 12, **dadurch gekennzeichnet, dass** das Molekulargewicht des hydrophilen makromolekularen Additivs mehr als 3 000 Dalton beträgt.

15. Verfahren nach Anspruch 13 oder 14, **dadurch gekennzeichnet, dass** das hydrophile makromolekulare Additiv ein hydrophiles Polymer mit einem Molekulargewicht von 3 000 bis 20 000 Dalton ist.

16. Verfahren nach einem der Ansprüche 13 bis 15, **dadurch gekennzeichnet, dass** das hydrophile makromolekulare Additiv Polyethylenglykol ist.

17. Verfahren nach einem der Ansprüche 13 bis 16, **dadurch gekennzeichnet, dass** das hydrophile makromolekulare Additiv aus Polysacchariden, insbesondere Stärke, Dextran bzw. Cellulose, oder Mucopolysacchariden ausgewählt ist.

18. Verfahren nach Anspruch 17, **dadurch gekennzeichnet, dass** das hydrophile makromolekulare Additiv ein Polysaccharid in oxidierter Form ist.

19. Verfahren nach einem der vorhergehenden Ansprüche, **dadurch gekennzeichnet, dass** die Collagenkomponente mit einer Dosis von 5 bis 50 kGy bestrahlt wird.

20. Verfahren nach einem der vorhergehenden Ansprüche, **dadurch gekennzeichnet, dass** die Collagenkomponente mit einer Dosis von 20 bis 50 kGy und vorzugsweise 25 bis 35 kGy bestrahlt wird.

21. Verfahren nach einem der vorhergehenden Ansprüche, **dadurch gekennzeichnet, dass** die Collagenkomponente mit β-Strahlung bestrahlt und das resultierende Collagenmaterial stark vernetzt wird und *in vivo* innerhalb von mehreren Wochen biologisch abbaubar ist.

22. Verfahren nach einem der vorhergehenden Ansprüche, **dadurch gekennzeichnet, dass** die Collagenkomponente vor ihrer Bestrahlung im feuchten Zustand mit einem Collagenfasernetz mit vorzugsweise Helixstruktur verbunden wird.

23. Verfahren nach Anspruch 22, **dadurch gekennzeichnet, dass** das Collagenfasernetz aus einem Umschlag aus zusammengepressten Fasern besteht, der aus einer sauren Lösung von nativem Collagen durch Behandlung mit Periodsäure oder einem ihrer Salze, Bildung von Fasern und anschließend Vernetzung durch Neutralisieren erhalten worden ist, wobei die vernetzten Collagenfasern mit Helixstruktur unter Druck zusammengepresst werden, **und dass** eine Lösung der Collagenkomponente auf den Umschlag aufgebracht und anschließend das Ganze mit β-Strahlung bestrahlt wird.

24. Zweikomponentiges Collagenverbundmaterial mit *in vivo* kontrollierbarer biologischer Abbaugeschwindigkeit, das biologisch verträglich, nicht-toxisch und steril ist und sich vernähen oder klammern lässt, **dadurch gekennzeichnet, dass** es ausschließlich oder hauptsächlich zwei Schichten umfasst, die innig miteinander verbunden und bei gegenseitiger Durchdringung der vernetzten Netzwerke vernetzt sind, wobei eine der Schichten von einem Film auf der Basis einer vernetzten Collagenkomponente und die andere von einem zusammengepressten Umschlag gebildet wird, der von unlöslich gemachten vernetzten Collagenfasern, insbesondere Collagen mit Helixstruktur, die aus in einer wässrigen Lösung solubilisiertem oder dispergiertem Collagen hergestellt sind, geformt wird.

25. Zweikomponentiges Collagenverbundmaterial nach Anspruch 24, **dadurch gekennzeichnet, dass** es durch das Verfahren nach Anspruch 22 oder 23 vernetzt worden ist.

26. Collagenmaterial nach Anspruch 24 oder 25, **dadurch gekennzeichnet, dass** die Collagenkomponente wie in einem der Ansprüche 7 bis 18 definiert ist.

## Claims

1. A process for preparing a crosslinked collagenic material which is biocompatible and nontoxic and has a controlled *in vivo* rate of biodegradation, **characterized in that** it comprises subjecting a collagenic component substantially free of any complementary crosslinking agent, and preferably not crosslinked, in the wet state to irradiation by beta rays, the collagenic material obtained being sterile and biodegradable over a few days to several weeks.

2. The process as claimed in claim 1, **characterized in that** the collagenic compound has a moisture content of greater than 30%, preferably greater than 40%.

3. The process as claimed in either of claims 1 and 2, **characterized in that** the collagenic component is in the form of a gel.

4. The process as claimed in either of claims 1 and 2, **characterized in that** the collagenic component is in the form of an aqueous solution.

5. The process as claimed in any one of the preceding claims, **characterized in that** the collagenic component has a neutral pH, preferably between 6.5 and 8.

6. The process as claimed in any one of the preceding claims, **characterized in that** the concentration of the collagenic component (solids contenc) is a minimum of 0.5% and preferably greater than 2.5%.

7. The process as claimed in any one of the preceding claims, **characterized in that** the collagenic component consists of or comprises collagen that has at least partially lost its helical structure.

8. The process as claimed in claim 7, **characterized in that** the collagen that has at least partially lost its helical structure is formed from unhydrolyzed collagen consisting mostly of α chains.

9. The process as claimed in either of claims 7 and 8, **characterized in that** the collagenic component consists of or comprises collagen that has at least partially lost its helical structure by heating above 37°C, preferably between 40 and 50°C.

10. The process as claimed in any one of claims 1 to 6, **characterized in that** the collagenic component consists of or comprises oxidized collagen.

11. The process as claimed in claim 10, **characterized in that** the oxidized collagen consists of collagen modified by oxidative scission using periodic acid or one of its salts.

12. The process as claimed in any one of claims 1 to 6, **characterized in that** the collagenic component comprises collagen functionalized at the level of the amino and/or carboxyl functional groups of the amino acids.

13. The process as claimed in any one of the preceding claims, **characterized in that** the collagenic component comprises a macromolecular hydrophilic additive.

14. The process as claimed in claim 12, **characterized in that** the macromolecular hydrophilic additive has a molecular weight greater than 3 000 daltons.

15. The process as claimed in either of claims 13 and 14, **characterized in that** the macromolecular hydrophilic additive is a hydrophilic polymer having a molecular weight of between 3 000 and 20 000 daltons.

16. The process as claimed in any one of claims 13 to 15, **characterized in that** the macromolecular hydrophilic additive is polyethylene glycol.

17. The process as claimed in any one of claims 13 to 16, **characterized in that** the macromolecular hydrophilic additive is chosen from polysaccharides, especially starch, dextran and cellulose, or mucopolysaccharides.

18. The process as claimed in claim 17, **characterized in that** the macromolecular hydrophilic additive is a polysaccharide in oxidized form.

19. The process as claimed in any one of the preceding claims, **characterized in that** the collagenic component is irradiated with a dose of 5 to 50 kGy.

20. The process as claimed in any one of the preceding claims, **characterized in that** the collagenic component is irradiated with a dose of 20 to 50 kGy, preferably 25 to 35 kGy.

21. The process as claimed in any one of the preceding claims, **characterized in that** the collagenic component is irradiated by beta rays, the resulting collagenic material being highly crosslinked and biodegradable *in vivo* over several weeks.

22. The process as claimed in any one of the preceding claims, **characterized in that** the collagenic component in the wet state is combined with a network of collagen fibers preferably of helical structure, prior to its irradiation.

23. The process as claimed in claim 22, **characterized in that** the network of collagen fibers consists of a compress of compacted fibers, obtained from an acid solution of native collagen, by treatment using periodic acid or one of its salts, formation of fibers and then crosslinking by neutralization, the resulting crosslinked collagen fibers of helical structure being compressed by applying pressure, and **in that** a solution of a collagenic component is deposited on said compress and then the assembly is irradiated by beta rays.

24. A collagenic bicomposite which is biocompatible, nontoxic and sterile, has a controlled *in vivo* rate of biodegradation and is able to be applied by sutures or staples, **characterized in that** it comprises only, or mainly, two layers intimately associated and crosslinked with interpenetration of the crosslinked networks, one of said layers being formed from a film based on a crosslinked collagenic component and the other from a compacted compress formed from crosslinked collagen fibers rendered insoluble, especially collagen fibers having a helical structure, prepared from collagen dissolved or dispersed in an aqueous solution.

25. The collagenic bicomposite as claimed in claim 24, **characterized in that** it is crosslinked by the process according to either of claims 22 and 23.

26. The collagenic bicomposite as claimed in either of claims 24 and 25, **characterized in that** the collagenic component is as defined in any one of claims 7 to 18.
